# EUROPEAN PATENT APPLICATION

(11) **EP 4 647 163 A1**
(43) Date of publication of application: **12.11.2025**
(21) Application number: 23914990.9
(22) Date of filing: 22.12.2023
(51) Int. Cl.: B01J 37/04, B01J 37/08, B01J 37/00, B01J 23/00, B01J 23/887, B01J 8/04, C07C 51/25, C07C 45/35, C07C 57/04

(54) **METHOD FOR PREPARING CATALYST FOR PREPARING (METH)ACRYLIC ACID, CATALYST FOR PREPARING (METH)ACRYLIC ACID, AND METHOD FOR PREPARING (METH)ACRYLIC ACID**

(30) Priority: 06.01.2023 KR 20230002291; 20.12.2023 KR 20230186928
(71) Applicant: LG Chem, Ltd., Yeongdeungpo-gu Seoul 07336 (KR)
(72) Inventor: KIM, Minsu, Daejeon 34122 (KR); LIM, Hyunsub, Daejeon 34122 (KR); CHOI, Byung Yul, Daejeon 34122 (KR); CHOE, Young Hyun, Daejeon 34122 (KR); CHOI, Youngju, Daejeon 34122 (KR); PARK, Hyun Woo, Daejeon 34122 (KR); SEO, Dong Woo, Daejeon 34122 (KR); LEE, Saeha, Daejeon 34122 (KR); LIM, Seulgi, Daejeon 34122 (KR); JOO, Hyeonho, Daejeon 34122 (KR)
(74) Representative: Goddar, Heinz J.
(86) International application number: PCT/KR2023/021360
(87) International publication number: WO 2024/147530

(57) **Abstract**

The present invention relates to a method for preparing a catalyst for preparing (meth)acrylic acid and a method for preparing (meth)acrylic acid.

## Description

### [Technical Field]

This application claims priority to and the benefit of Korean Patent Application Nos. 10-2023-0002291 and 10-2023-0186928 filed in the Korean Intellectual Property Office on January 6, 2023 and December 20, 2023, respectively, the entire contents of which are incorporated herein by reference.

The present application relates to a method for preparing a catalyst for preparing (meth)acrylic acid, a catalyst for preparing (meth)acrylic acid, and a method for preparing (meth)acrylic acid.

### [Background Art]

Generally, for the preparation of (meth)acrylic acid, gas-phase contact oxidization methods of propylene, isobutylene, and the like with molecular oxygen in a fixed bed multitubular reactor in which a catalyst layer is present have been used.

The above reaction is divided into a first-stage reaction zone where propylene, isobutylene, and the like are partially oxidized to prepare (meth)acrolein, and a second-stage reaction zone where the (meth) acrolein is partially oxidized to prepare (meth) acrylic acid.

The first-stage reaction zone and the second-stage reaction zone are provided with a first catalyst layer and a second catalyst layer, respectively, and the catalyst layer may include a catalyst for preparing (meth)acrylic acid. Further, a molybdenum-bismuth-based composite metal oxide is used as a catalyst for preparing the (meth)acrylic acid.

Generally, the molybdenum-bismuth-based composite metal oxide is prepared by a method of preparing a suspension including a precursor compound of each metal element, co-precipitating the suspension, and calcinating the resulting product, and nitrate or ammonium salt is usually used as the precursor compound.

Since there is a problem in that nitrate reduces the drying efficiency of the suspension in this process, the type of precursor needs to be changed. In addition, there are a problem in dealing with ammonia and NOx materials generated by using the nitrates and ammonium salts, a equipment corrosion problem caused by these materials, a radiation exposure problem for workers during work, and the like, so that there is a need for a method capable of solving these problems.

[Citation List] (Patent Document 1) Korean Patent Application Laid-Open No. 10-2014-0138081

### [Detailed Description of the Invention]

### [Technical Problem]

The present application has been made in an effort to provide a method for preparing a catalyst for preparing (meth)acrylic acid that has excellent suspension drying efficiency and reduces the amount of nitric acid or ammonia generated during the preparation process, a catalyst for preparing (meth)acrylic acid, and a method for preparing (meth)acrylic acid using the catalyst.

### [Technical Solution]

An exemplary embodiment of the present application provides a method for preparing a catalyst for preparing (meth)acrylic acid, the method including:
preparing a first catalyst suspension including a molybdenum-based compound; a bismuth-based compound; an M²-based compound; an M³-based first compound; and an M⁶-based compound;
preparing a second catalyst suspension by adding an M³-based second compound to the first catalyst suspension; and
preparing a molybdenum-bismuth-based composite metal oxide represented by the following Chemical Formula 1 by drying the second catalyst suspension, in which the M³-based first compound is one selected from the group consisting of M³-based metal nitrates, acetates, chlorides, sulfates, and mixtures thereof,
the M³-based second compound is one selected from the group consisting of M³-based metal organic acid salts having 6 or more carbon atoms, carbonates, hydroxides, oxides, and mixtures thereof, and the second catalyst suspension includes a soluble M³-based compound and an insoluble M³-based compound.

   [Chemical Formula 1] MoₐBi_{b}M¹_{c}M²_{d}M³ₑM⁴_{f}M⁵_{g}M⁶ₕOᵢ
In Chemical Formula 1,
Mo is molybdenum,
Bi is bismuth,
M¹ is one or more elements selected from the group consisting of V, W, P, As, Sb, S, Se, Te, Cd, and Pb,
M² is one or more elements selected from the group consisting of Fe, Cu, Zn, Cr, and Mn,
M³ is one or more elements selected from the group consisting of Co, Ni, Nb, Ta, Ga, In, Ge, and Sn,
M⁴ is one or more elements selected from the group consisting of Si, Al, Ti, Zr, and Ce,
M⁵ is one or more elements selected from the group consisting of Au, Pd, Pt, Ir, Ru, Ag, and Rh,
M⁶ is one or more elements selected from the group consisting of Na, K, Li, Rb, Cs, Ca, Mg, Sr, and Ba,
a, b, c, d, e, f, g, h, and i represent the atomic proportion of each element,
when a is 12, b is 0.01 to 20, c is 0 to 20, d is 0.001 to 15, e is 0.001 to 20, and f is 0 to 20,
g is 0 to 10, h is 0.001 to 10, and i is a numerical value determined by the oxidation state of each of the components.

Another exemplary embodiment of the present application provides a catalyst for preparing (meth)acrylic acid prepared by the method for preparing a catalyst for preparing (meth)acrylic acid.

A final exemplary embodiment of the present application provides a method for preparing (meth)acrylic acid using a fixed bed reactor including a front catalyst layer and a rear catalyst layer, the method including: preparing (meth)acrolein by supplying an injection gas including a raw material gas and oxygen gas to the front catalyst layer; and preparing (meth)acrylic acid by supplying the (meth)acrolein to the rear catalyst layer, in which the catalyst for preparing (meth)acrylic acid is included in at least one of the front catalyst layer and the rear catalyst layer.

### [Advantageous Effects]

Since the method for preparing a catalyst for preparing (meth)acrylic acid according to an exemplary embodiment of the present application can improve the drying efficiency of a suspension, the time taken to prepare the catalyst can be reduced.

The method for preparing a catalyst for preparing (meth)acrylic acid according to an exemplary embodiment of the present invention can reduce the amount of nitric acid and ammonia generated during the preparation of the catalyst.

When using the catalyst for preparing (meth)acrylic acid prepared by the preparation method according to an exemplary embodiment of the present invention, the yield of (meth)acrylic acid can be increased.

### [Best Mode]

Hereinafter, the present specification will be described in more detail.

When one member is disposed "on" another member in the present specification, this includes not only a case where the one member is brought into contact with another member, but also a case where still another member is present between the two members.

When one part "includes" one constituent element in the present specification, unless otherwise specifically described, this does not mean that another constituent element is excluded, but means that another constituent element may be further included.

In the present specification, "p to q" means "p or more and q or less", and thus, includes p and q.

In the present specification, "p~q" means "p or more and q or less", and thus, includes p and q.

In the present specification, "(meth)acrylic acid" means acrylic acid or methacrylic acid.

### <Method for preparing catalyst for preparing (meth)acrylic acid>

Hereinafter, a method for preparing a catalyst for preparing (meth)acrylic acid will be described.

An exemplary embodiment of the present application provides a method for preparing a catalyst for preparing (meth)acrylic acid, the method including: preparing a first catalyst suspension including a molybdenum-based compound; a bismuth-based compound; an M²-based compound; an M³-based first compound; and an M⁶-based compound; preparing a second catalyst suspension by adding an M³-based second compound to the first catalyst suspension; and preparing a molybdenum-bismuth-based composite metal oxide represented by the following Chemical Formula 1 by drying the second catalyst suspension, in which the M³-based first compound is one selected from the group consisting of M³-based metal nitrates, acetates, chlorides, sulfates, and mixtures thereof, the M³-based second compound is one selected from the group consisting of M³-based metal organic acid salts having 6 or more carbon atoms, carbonates, hydroxides, oxides, and mixtures thereof, and the second catalyst suspension includes a soluble M³-based compound and an insoluble M³-based compound.

[Chemical Formula 1] MoₐBi_{b}M¹_{c}M²_{d}M³ₑM⁴_{f}M⁵_{g}M⁶ₕOᵢ

Mo is molybdenum,
Bi is bismuth,
M¹ is one or more elements selected from the group consisting of V, W, P, As, Sb, S, Se, Te, Cd, and Pb,
M² is one or more elements selected from the group consisting of Fe, Cu, Zn, Cr, and Mn,
M³ is one or more elements selected from the group consisting of Co, Ni, Nb, Ta, Ga, In, Ge, and Sn,
M⁴ is one or more elements selected from the group consisting of Si, Al, Ti, Zr, and Ce,
M⁵ is one or more elements selected from the group consisting of Au, Pd, Pt, Ir, Ru, Ag, and Rh,
M⁶ is one or more elements selected from the group consisting of Na, K, Li, Rb, Cs, Ca, Mg, Sr, and Ba,
a, b, c, d, e, f, g, h, and i represent the atomic proportion of each element,
when a is 12, b is 0.01 to 20, c is 0 to 20, d is 0.001 to 15, e is 0.001 to 20, and f is 0 to 20,
g is 0 to 10, h is 0.001 to 10, and i is a numerical value determined by the oxidation state of each of the components.

The method for preparing a catalyst for preparing (meth)acrylic acid according to the present application is characterized by preparing a second catalyst suspension by adding an M³-based second compound which is one selected from the group consisting of M³-based metal organic acid salts having 6 or more carbon atoms, carbonates, hydroxides, oxides, and mixtures thereof to a first catalyst suspension including an M³-based first compound which is one selected from the group consisting of M³-based metal nitrates, acetates, chlorides, sulfates, and mixtures thereof, and through this, the drying efficiency of the catalyst suspension may be increased. As a result, the catalyst preparation time may be shortened.

In the present specification, when a given metal is represented by M and n is a value calculated as the oxidation number of M when the given metal forms a salt with a certain counter anion, the nitrate of the metal may be represented by M(NO₃)n, the acetate of the metal may be represented by (CH₃COO)nM, the chloride of the metal may be represented by M(Cl)n, the sulfate of the metal may be represented by M(SO₄)n, the organic acid salt having 6 or more carbons of the metal may be represented by (CH₃(CH₂)₁₀COO)nM (for example, when the paired anion of the organic acid salt is C6 or more laurate), the carbonate of the metal may be represented by M(CO₃)n, the hydroxide of the metal may be represented by M(OH)n, and the oxide of the metal may be represented by M(O)n, and in some cases, the salt may be present in the form of hydroxide.

By additionally adding the M³-based second compound in the form of oxide or hydroxide, there is also an effect capable of preventing a phenomenon in which a large amount of ammonia and NOx materials generated by nitrates and ammonium salts are discharged.

Furthermore, the method for preparing a catalyst for preparing (meth)acrylic acid according to the present application is characterized in that the second catalyst suspension includes a soluble M³-based compound and an insoluble M³-based compound, and when using a catalyst for preparing (meth)acrylic acid prepared by the preparation method according to an exemplary embodiment of the present invention, the yield of (meth)acrylic acid may be increased.

In an exemplary embodiment of the present application, Chemical Formula 1 may be represented by the following Chemical Formula 2.

Mo₁₂Bi_{1.5}Fe_{3.0}M³_{8.0}Cs_{0.8}

In Chemical Formula 2,
M³ is one or more elements selected from the group consisting of Co, Ni, Nb, Ta, Ga, In, Ge, and Sn.

In an exemplary embodiment of the present invention, the method for preparing a catalyst for preparing (meth)acrylic acid includes: preparing a first catalyst suspension including a molybdenum-based compound; a bismuth-based compound; an M²-based compound; an M³-based first compound; and an M⁶-based compound; and preparing a second catalyst suspension by adding an M³-based second compound to the first catalyst suspension. The preparing of the first catalyst suspension and the second catalyst suspension may be performed by a method of dissolving or dispersing the compounds in a solvent such as water as a known method.

In an exemplary embodiment of the present invention, the method for preparing a catalyst for preparing (meth)acrylic acid includes drying the second catalyst suspension. The drying step described above is a step for forming a solid by evaporating the water included in the second catalyst suspension. The drying method is not particularly limited and it is possible to apply, for example, a method of drying using a spray dryer, a method of drying using a slurry dryer, a method of drying using a drum dryer, a method of drying by evaporation, a method of drying using an oven, and the like.

In an exemplary embodiment of the present invention, the drying of the second catalyst suspension may be performed at a temperature of 60°C to 200°C and under air conditions. The temperature may be adjusted to a temperature of 100°C to 190°C or a temperature of 120°C to 180°C. When the temperature does not reach the above temperature range, drying may not be sufficiently performed, and when the temperature exceeds the above temperature range, the catalyst may be damaged.

In an exemplary embodiment of the present invention, the drying of the second catalyst suspension may be performed for a time of 1 h to 48 h. The time may be adjusted to a time of 5 h to 40 h or a time of 10 h to 30 h. The h means time, and when the h does not reach the above time range, drying may not be sufficiently performed, and when the h exceeds the above time range, the catalyst may be damaged.

In an exemplary embodiment of the present invention, the drying of the second catalyst suspension may be performed by a method of placing the catalyst suspension in an oven and adjusting the temperature and time.

In an exemplary embodiment of the present invention, the molybdenum-based compound; the bismuth-based compound; the M²-based compound; the M³-based first compound; the M³-based second compound; and the M⁶-based compound may be in the form of a precursor including each metal element.

In particular, in an exemplary embodiment of the present invention, the M³-based first compound may be one selected from the group consisting of M³-based metal nitrates, acetates, sulfates, chlorides, and mixtures thereof, and the M³-based second compound may be one selected from the group consisting of M³-based metal organic acid salts having 6 or more carbon atoms, carbonates, hydroxides, oxides, and mixtures thereof.

That is, the method is characterized by adding a precursors in forms (that is, M³-based metal organic acid salts having 6 or more carbon atoms, carbonates, hydroxides, oxides, and mixtures thereof) other than nitrate precursors in the process of preparing the second catalyst suspension, and the drying efficiency of the catalyst suspension may be increased through this addition.

In an exemplary embodiment of the present invention, the M³-based second compound may be an M³-based metal oxide or hydroxide. The present invention has an advantage in that by using the M³-based second compound in the form of the M³-based metal oxide or hydroxide, the suspension drying efficiency is increased and simultaneously, the generation of nitric acid is further minimized compared to when a precursor in the form of nitrate is used, or the generation of ammonia is further minimized compared to when a precursor in the form of an ammonium salt is used.

Further, in an exemplary embodiment of the present invention, the amount (g) or number (mol) of moles of the generated nitric acid or ammonia may be calculated from the stoichiometric relationship between nitric acid and ammonia included in each metal precursor. For example, ammonium molybdate ({ (NH₄)₆(Mo₇O₂₄)·4H₂O}) is used as a molybdenum-based compound, the amount (g) of ammonia generated may be calculated in consideration of the mole number and molecular weight of ammonium molybdate. Specifically, the amount of ammonia (g) = {mass of ammonium molybdate/molecular weight of ammonium molybdate} x {mole number ratio of ammonia included in ammonium molybdate} x (molecular weight of ammonia). Specifically, 1,000 g of ammonium molybdate ((NH₄)₆(Mo₇O₂₄)·4H₂O) is used, the amount (g) of ammonia generated is calculated as {(1,000 g)/(1235.9 g/mol)} x (6 mol/1 mol) x (18 g/mol) = 87.39 g.

In an exemplary embodiment of the present invention, the oxide or hydroxide means the oxide or hydroxide form of each metal.

In an exemplary embodiment of the present invention, the molybdenum-based compound may be ammonium molybdate [(NH₄)₆Mo₇O₂₄].

In an exemplary embodiment of the present invention, the bismuth-based compound may be bismuth nitrate [Bi(NO₃)₃].

In an exemplary embodiment of the present invention, based on the total weight of the catalyst suspension, the total weight of the oxide or hydroxide of each element in the molybdenum-based compound; the bismuth-based compound; the M²-based compound; the M³-based compound; and the M⁶-based compound may be 1 wt% or more and 100 wt% or less.

In an exemplary embodiment of the present invention, the second catalyst suspension may include the soluble M³-based compound and the insoluble M³-based compound at a weight ratio of 1:6 to 6:1, preferably 1:3 to 3:1, more preferably 1:1. When the above weight ratio is satisfied, the catalyst performance may be further improved, and the closer the weight ratio is to 1:1, the more the process may be further improved.

For example, M³ in the soluble M³-based compound and the insoluble M³-based compound may be Co or Ni, which is the same as each other, but is not limited thereto. That is, examples of the soluble M³-based compound and the insoluble M³-based compound each include a soluble cobalt salt (for example: Co(NO₃)₂·6H₂O) and an insoluble cobalt salt (for example: Co(OH)₂), or a soluble nickel salt (for example: Ni(NO₃)₂·6H₂O) and an insoluble nickel salt (for example: NiO).

The method for preparing a catalyst for preparing (meth)acrylic acid according to an exemplary embodiment of the present invention further includes heating the second catalyst suspension. The second catalyst suspension may be reacted by the heating of the second catalyst suspension. A product produced by the reaction is meant to include a molybdenum-based compound; a bismuth-based compound; an M²-based compound; an M³-based first compound; an M³-based second compound; and an M⁶-based compound in the form of a slurry.

The method for preparing a catalyst for preparing (meth)acrylic acid according to an exemplary embodiment of the present invention further includes heating and drying the second catalyst suspension. Through this process, the molybdenum-bismuth-based composite metal oxide represented by Chemical Formula 1 may be prepared.

The method for preparing a catalyst for preparing (meth)acrylic acid according to an exemplary embodiment of the present invention further includes pulverizing the molybdenum-bismuth-based composite metal oxide represented by Chemical Formula 1.

In an exemplary embodiment of the present invention, the molybdenum-bismuth-based composite metal oxide may be pulverized to an average particle diameter of 10 µm to 1,000 µm.

The method for preparing a catalyst for preparing (meth)acrylic acid according to an exemplary embodiment of the present invention further includes mixing the molybdenum-bismuth-based composite metal oxide represented by Chemical Formula 1 and a binder. The binder is introduced to improve the strength and abrasion resistance of the catalyst.

In an exemplary embodiment of the present invention, as the binder, an organic-based binder or an inorganic-based binder may be used. Examples of the corresponding organic-based binder include polymer compounds such as polyvinyl alcohol, α-glucan derivatives, β-glucan derivatives, and the like. These may be used alone or in combination of two or more thereof.

The α-glucan derivative refers to a polysaccharide in which glucose is bound in an α-type structure among polysaccharides composed of glucose, and examples thereof include derivatives such as α1-4 glucan, α1-6 glucan, and α1-4/1-6 glucan. Specific examples of such α-glucan derivatives include amylose, glycogen, amylopectin, pullulan, dextrin, cyclodextrin, and the like. These may be used alone or in combination of two or more thereof.

The β-glucan derivative refers to a polysaccharide in which glucose is bound in a β-type structure among polysaccharides composed of glucose, and examples thereof include derivatives such as β1-4 glucan, β1-3 glucan, β1-6 glucan, and β1-3/1-6 glucan. Examples of such β-glucan derivatives include cellulose derivatives such as methylcellulose, ethylcellulose, carboxymethylcellulose, sodium carboxymethylcellulose, hydroxyethylcellulose, hydroxypropylcellulose, hydroxypropylmethylcellulose, hydroxyethylmethylcellulose, hydroxybutylmethylcellulose, ethylhydroxyethylcellulose, and hydroxypropylcellulose, β1-3 glucans such as curdlan, laminaran, paramylon, callose, pachyman and scleroglucan, and the like. These may be used alone or in combination of two or more thereof.

Although the organic-based binder may be used in a crude or purified state, it is desirable that the content of metal impurities or residue on ignition be as low as possible in order to suppress the deterioration of catalyst performance due to metal or residue on ignition as impurities.

Examples of the inorganic-based binder include an inert carrier such as an inorganic compound (or an inorganic fiber) such as silica, alumina, silica-alumina, silicon carbide, titania, magnesia, graphite, and diatomaceous earth, a ceramic ball, stainless steel, and an inorganic fiber such as glass fiber, ceramic fiber, and carbon fiber. These may be used alone or in combination of two or more thereof. Further, the organic-based binder and the inorganic-based binder may also be used in mixture.

The amount of binder used is appropriately selected depending on the type or size of the catalyst precursor, the type of liquid, and the like, but is preferably 0.05 parts by mass to 15 parts by mass, more preferably 0.1 parts by mass to 10 parts by mass, and even more preferably 1 part by mass to 8 parts by mass based on 100 parts by mass of the catalyst precursor.

In addition, additives widely known as powder binders such as ammonium nitrate (pore forming agent), cellulose, starch, polyvinyl alcohol, and stearic acid may be used as the type of binder.

In an exemplary embodiment of the present invention, the first catalyst suspension and the second catalyst suspension may each include a solvent, and examples of the solvent include water, alcohol (particularly, ethyl alcohol), acetone, and the like. These may be used alone or in combination of two or more thereof. Among them, it is preferred to use a mixed solution of water and alcohol.

The method for preparing a catalyst for preparing (meth)acrylic acid according to an exemplary embodiment of the present invention further includes supporting the molybdenum-bismuth-based composite metal oxide represented by Chemical Formula 1 on a carrier.

In an exemplary embodiment of the present invention, the supporting of the molybdenum-bismuth-based composite metal oxide represented by Chemical Formula 1 on the carrier may be performed using a method of mixing the molybdenum-bismuth-based composite metal oxide represented by Chemical Formula 1 and the carrier.

In an exemplary embodiment of the present invention, the structure of a material for the carrier is not particularly limited as long as the material does not inhibit the activity of the catalyst. For example, the carrier may be one or more compounds selected from the group consisting of SiO₂, Al₂O₃, MgO, MgCl₂, CaCl₂, ZrO₂, TiO₂, B₂O₃, CaO, ZnO, BaO, ThO₂, SiO₂-Al₂O₃, SiO₂-MgO, SiO₂-TiO₂, SiO₂-V₂O₅, SiO₂-CrO₂O₃, SiO₂-TiO₂-MgO, and zeolite.

The method for preparing a catalyst for preparing (meth)acrylic acid according to an exemplary embodiment of the present invention may further include calcinating the molybdenum-bismuth-based composite metal oxide represented by Chemical Formula 1.

The method for preparing a catalyst for preparing (meth)acrylic acid according to an exemplary embodiment of the present invention may further include performing calcinating at 350°C to 650°C for 1 hour (h) to 10 hours, preferably at 400°C to 600°C for 5 hours to 10 hours. Preferably, the calcinating may be performed in an inert gas or oxidizing atmosphere (for example, air or a mixed gas atmosphere of an inert gas and oxygen), and another reducing atmosphere (for example, a mixed gas atmosphere of an inert gas, oxygen and NH₃, CO and/or H₂). Furthermore, the calcinating time may be several minutes to several hours, and may be generally shortened as the temperature increases.

In some cases, after pulverization is performed, the above-described additive, binder, and solvent may be added and extrusion molded, and then calcinating may be performed.

### <Catalyst for preparing (meth)acrylic acid>

Hereinafter, a catalyst for preparing (meth)acrylic acid will be described.

An exemplary embodiment of the present invention provides a catalyst for preparing (meth)acrylic acid prepared by the above-described method for preparing a catalyst for preparing (meth)acrylic acid.

The catalyst for preparing (meth)acrylic acid according to an exemplary embodiment of the present invention may increase the yield of (meth)acrylic acid when used in a reaction.

### <Method for preparing (meth)acrylic acid>

Hereinafter, a method for preparing (meth)acrylic acid will be described.

An exemplary embodiment of the present application provides a method for preparing (meth)acrylic acid using a fixed bed reactor including a front catalyst layer and a rear catalyst layer, the method including: preparing (meth)acrolein by supplying an injection gas including a raw material gas and oxygen gas to the front catalyst layer; and preparing (meth)acrylic acid by supplying the (meth)acrolein to the rear catalyst layer, in which the above-described catalyst for preparing (meth)acrylic acid is included in at least one of the front catalyst layer and the rear catalyst layer.

In an exemplary embodiment of the present invention, the raw material gas may include one selected from the group consisting of propylene, isobutylene, t-butyl alcohol, and methyl-t-butyl ether, or a mixture thereof.

In an exemplary embodiment of the present invention, the raw material gas may include propylene, isobutylene, or a mixture thereof.

In an exemplary embodiment of the present invention, the raw material gas may include propylene.

Except for using the catalyst according to the present application, methods typically used for the preparation of (meth)acrylic acid may be applied.

### [Mode for Invention]

Hereinafter, the present application will be described in detail with reference to Examples for specifically describing the present application. However, the Examples according to the present application may be modified in various forms, and it is not interpreted that the scope of the present application is limited to the Examples described in detail below. The Examples of the present application are provided for more completely explaining the present application to the person with ordinary skill in the art.

### Examples.

### <Example 1>

A first solution was prepared by putting 1,500 ml of distilled water into a 5-L reactor equipped with a stirrer and dissolving 500 g of (NH₄)₆Mo₇O₂₄·4H₂O. Apart from this, a second solution was prepared by putting 171 g of Bi(NO₃)₃·5H₂O, 90 g of Fe(NO₃)₃·9H₂O, 270g of Co(NO₃)₂·6H₂O, and 40 g of CsNO₃ were dissolved in 500 ml of distilled water and dissolving the resulting mixture. A first catalyst suspension was prepared by mixing the first solution and the second solution for 30 minutes. Thereafter, a second catalyst suspension was prepared by adding 90 g of Co(OH)₂ to the first catalyst suspension. The prepared second catalyst suspension was put into an electric oven at 160°C and dried for 20 hours, and then pulverized to a particle diameter of about 200 µm.

After adding 130 g of a pore forming agent (ammonium nitrate), 5 wt% of inorganic fibers (Cerakwool, silica-alumina fiber, manufactured by KCC Corporation) and 20 wt% of a mixed solution of water and alcohol were added to the pulverized powder and re-mixed, the resulting mixture was extrusion molded to a size of 5 to 8 mm. The molded catalyst was fired by performing heat treatment at a temperature of 530°C for 5 hours.

It was confirmed that the composition ratio of the elements other than oxygen among the catalyst active components was Mo₁₂Bi_{1.5}Fe_{3.0}Co_{8.0}Cs_{0.8}.

### <Example 2>

A catalyst was prepared in the same manner as in Example 1, such that the composition ratio of the elements other than oxygen among the catalyst active components was Mo₁₂Bi_{1.5}Fe_{3.0}Co_{8.0}Cs_{0.8}, except that 412 g of Co(NO₃)₂·6H₂O was used and 45 g of Co(OH)₂ was used in Example 1.

### <Example 3>

A catalyst was prepared in the same manner as in Example 1 such that the composition ratio of the elements other than oxygen among the catalyst active components was Mo₁₂Bi_{1.5}Fe_{3.0}Co_{8.0}Cs_{0.8}, except that 137g of Co(NO₃)₂·6H₂O was used and 134g of Co(OH)₂ was used in Example 1.

### <Example 4>

A catalyst was prepared in the same manner as in Example 1 such that the composition ratio of the elements other than oxygen among the catalyst active components was Mo₁₂Bi_{1.5}Fe_{3.0}Co_{8.0}Cs_{0.8}, except that 473g of Co(NO₃)₂·6H₂O was used and 24g of Co(OH)₂ was used in Example 1.

### <Example 5>

A catalyst was prepared in the same manner as in Example 1, such that the composition ratio of the elements other than oxygen among the catalyst active components was Mo₁₂Bi_{1.5}Fe_{3.0}Co_{8.0}Cs_{0.8}, except that 75g of Co(NO₃)₂·6H₂O was used and 154g of Co(OH)₂ was used in Example 1.

### <Example 6>

A catalyst was prepared in the same manner as in Example 1, such that the composition ratio of the elements other than oxygen among the catalyst active components was Mo₁₂Bi_{1.5}Fe_{3.0}Co_{8.0}Cs_{0.8}, except that Co(OH)₂ was replaced with 115 g of CoCO₃ in Example 1.

### <Example 7>

A catalyst was prepared in the same manner as in Example 1, such that the composition ratio of the elements other than oxygen among the catalyst active components was Mo₁₂Bi_{1.5}Fe_{3.0}Ni_{8.0}Cs_{0.8}, except that Co(NO₃)₂·6H₂O was replaced with 290 g of Ni(NO₃)₂·6H₂O and Co(OH)₂ was replaced with 75 g of NiO in Example 1.

### <Example 8>

A catalyst was prepared in the same manner as in Example 7, such that the composition ratio of the elements other than oxygen among the catalyst active components was Mo₁₂Bi_{1.5}Fe_{3.0}Ni_{8.0}Cs_{0.8}, except that 425 g of Ni(NO₃)₂·6H₂O was used and 36 g of NiO was used in Example 7.

### <Example 9>

A catalyst was prepared in the same manner as in Example 7, such that the composition ratio of the elements other than oxygen among the catalyst active components was Mo₁₂Bi_{1.5}Fe_{3.0}Ni_{8.0}Cs_{0.8}, except that 142 g of Ni(NO₃)₂·6H₂O was used and 109 g of NiO was used in Example 7.

### <Example 10>

A catalyst was prepared in the same manner as in Example 7, such that the composition ratio of the elements other than oxygen among the catalyst active components was Mo₁₂Bi_{1.5}Fe_{3.0}Ni_{8.0}Cs_{0.8}, except that 490 g of Ni(NO₃)₂·6H₂O was used and 20 g of NiO was used in Example 7.

### <Example 11>

A catalyst was prepared in the same manner as in Example 7, such that the composition ratio of the elements other than oxygen among the catalyst active components was Mo₁₂Bi_{1.5}Fe_{3.0}Ni_{8.0}Cs_{0.8}, except that 80 g of Ni(NO₃)₂·6H₂O was used and 125 g of NiO was used in Example 7.

### <Comparative Example 1>

A catalyst was prepared in the same manner as in Example 1, such that the composition ratio of the elements other than oxygen among the catalyst active components was Mo₁₂Bi_{1.5}Fe_{3.0}Co_{8.0}Cs_{0.8}, except that 549 g of Co(NO₃)₂·6H₂O was used and Co(OH)₂ was not used in Example 1.

### <Comparative Example 2>

A catalyst was prepared in the same manner as in Example 1, such that the composition ratio of the elements other than oxygen among the catalyst active components was Mo₁₂Bi_{1.5}Fe_{3.0}Co_{8.0}Cs_{0.8}, except that Co(NO₃)₂·6H₂O was not used and 179 g of Co(OH)₂ was used in Example 1.

### <Comparative Example 3>

A catalyst was prepared in the same manner as in Example 1, such that the composition ratio of the elements other than oxygen among the catalyst active components was Mo₁₂Bi_{1.5}Fe_{3.0}Ni_{8.0}Cs_{0.8}, except that Co(NO₃)₂·6H₂O was replaced with 566 g of Ni(NO₃)₂·6H₂O and Co(OH)₂ was not used in Example 1.

### <Comparative Example 4>

A catalyst was prepared in the same manner as in Example 1, such that the composition ratio of the elements other than oxygen among the catalyst active components was Mo₁₂Bi_{1.5}Fe_{3.0}Ni_{8.0}Cs_{0.8}, except that Co(NO₃)₂·6H₂O was not used and Co(OH)₂ was replaced with 145 g of NiO in Example 1.

### <Experimental Examples: Catalyst activity test>

A pilot-scale shell-and-tube type fixed bed reactor was prepared, which was composed of a steel reaction tube with an inner diameter of 1 inch and a fixed bed packed section length of 3000 mm, and a shell (diameter 100 mm) that covers the reaction tube and allows a heat medium to flow. The reactor included a front catalyst layer or a rear catalyst layer, and the front catalyst layer or the rear catalyst layer was each filled with the catalysts of Examples 1 to 11 and Comparative Examples 1 to 4. Thereafter, the raw material mixed gas (7.5 vol% raw gas, 14 vol% oxygen, 18 vol% water vapor, and 60.5 vol% inert nitrogen gas) was flowed at a space velocity of 80 hr⁻¹ to perform the reaction at a reaction temperature of 310°C. The conversion rate and selectivity were calculated by comparing the number of moles of each produced material using a gas chromatography analyzer for the gas that passed through the reactor.

As the raw material gas mixture (raw material gas), one of propylene, isobutylene, t-butyl alcohol, and methyl-t-butyl ether, or a mixture thereof may be used, but in this experimental example, an experiment was conducted when the raw material gas included propylene and isobutylene.

### - Gas chromatography operating conditions

Instrument: Agilent 7890B Gas Chromatography system
Column: Molecular sieve 5A 2.1 mm (L.D)*2.0 M 80/100 mesh
Plot Q 2.1 mm (I.D)*2.0 M 80/100 mesh
WAX-DA 0.53 mm (I.D)*30 M*1 µm
Solvent: 1,4-dioxane
Column temperature: measured while increasing the temperature from 50°C to 200°C
Moisture content: measured at 120°C for 10 minutes using an MX-50 moisture content apparatus

The conversion rate of the raw material gas (propylene and isobutylene), the selectivity of the unsaturated aldehyde and unsaturated acid produced, and the like were calculated, and are shown in the following Table 1. Conversion rate (%) = [(the number of moles of raw material gas reacted)/(the number of moles of raw material gas supplied)] x 100 (%) Selectivity (%) = [(the number of moles of unsaturated aldehyde and unsaturated acid produced)/(the number of moles of raw material gas reacted)] x 100 (%) Yield (%) = (the number of moles of produced unsaturated aldehyde and unsaturated acid)/the number of moles of raw material gas supplied × 100(%) Drying time (h) = time taken for the moisture content of the dry catalyst slurry to reach 10%

**[Table 1]**

| No. | Soluble M³ : Insoluble M³ | Conversion rate (%) | Selectivity (%) | Yield (%) | Drying time (h) |
|---|---|---|---|---|---|
| Example 1 | 1:1 | 95.5 | 87.9 | 84.0 | 20 |
| Example 2 | 3:1 | 95.0 | 86.3 | 82.0 | 30 |
| Example 3 | 1:3 | 96.0 | 85.4 | 82.0 | 18 |
| Example 4 | 6:1 | 90.0 | 84.4 | 76.0 | 36 |
| Example 5 | 1:6 | 91.0 | 83.5 | 75.9 | 16.5 |
| Example 6 | 1:1 | 95.0 | 87.8 | 83.4 | 20 |
| Example 7 | 1:1 | 95.0 | 87.4 | 83.0 | 20 |
| Example 8 | 3:1 | 93.5 | 85.6 | 80.0 | 31 |
| Example 9 | 1:3 | 97.0 | 85.2 | 80.1 | 18 |
| Example 10 | 6:1 | 88.5 | 85.0 | 75.2 | 36 |
| Example 11 | 1:6 | 89.5 | 84.3 | 75.4 | 16.5 |
| Comparative Example 1 | 10:0 | 87.0 | 83.8 | 72.9 | 48 |
| Comparative Example 2 | 0:10 | 88.0 | 79.5 | 70.0 | 16 |
| Comparative Example 3 | 10:0 | 75.0 | 85.0 | 63.8 | 48 |
| Comparative Example 4 | 0:10 | 87.0 | 81.0 | 63.2 | 16 |

In Table 1 above, soluble M³: insoluble M³ means the weight ratio of the soluble M³-based compound and the insoluble M³-based compound, the conversion rate means the conversion rate (%) of the raw material gas (propylene and isobutylene), and the selectivity means the selectivity (%) of unsaturated aldehyde and unsaturated acid. In this case, the unsaturated aldehyde and unsaturated acid may be (meth)acrolein and (meth)acrylic acid, respectively.

In Table 1 above, the yield means the yield of (meth)acrylic acid, and the drying time means the time taken for the moisture content of the dry catalyst slurry to reach 10%.

As can be confirmed in Table 1 above, it could be confirmed that the catalysts of Examples 1 to 11 prepared by the method for preparing a catalyst for preparing (meth)acrylic acid according to the present application had high drying efficiency of the catalyst suspension while having higher conversion rates and yields than those of the catalysts of Comparative Examples 1 to 4. That is, it could be confirmed that a catalyst with a high conversion rate could be prepared while shortening the preparation time of the catalyst.

In particular, it could be confirmed that among Examples 1 to 11, when the catalysts of Examples 1 to 3 and 6 to 9 were used, the yield of (meth)acrylic acid was even higher.

It could be confirmed that Comparative Examples 1 and 3, in which only the soluble M³-based compound was included in the second catalyst suspension, exhibited the selectivities at a level similar to those of the Examples, but were not better than the Examples in terms of other conversion rate, yield, and drying time. In particular, it could be confirmed that the drying time was the worst.

In addition, it could be confirmed that Comparative Examples 2 and 4, in which only the insoluble M³-based compound was included in the second catalyst suspension, were excellent in terms of drying time, but were not better than the Examples in terms of yield and selectivity parts. In addition, it could be confirmed that Comparative Examples 2 and 4 were not better than Comparative Examples 1 and 3 in terms of yield and selectivity.

That is, from the results in Table 1 above, it could be confirmed that the catalysts of Examples 1 to 11 prepared by the method for preparing a catalyst for preparing (meth)acrylic acid according to the present application had excellent performance, and among them, Examples 1 to 3 and 6 to 9 had the best performance. Furthermore, the method for preparing a catalyst for preparing (meth)acrylic acid according to the present application also has an effect capable of preventing a phenomenon in which a large amount of ammonia and NOx materials generated by nitrates and ammonium salts are discharged by adding an oxide or an M³-based compound in the form of an oxide.

## Claims

1. A method for preparing a catalyst for preparing (meth)acrylic acid, the method comprising:
preparing a first catalyst suspension comprising a molybdenum-based compound; a bismuth-based compound; an M²-based compound; an M³-based first compound; and an M⁶-based compound;
preparing a second catalyst suspension by adding an M³-based second compound to the first catalyst suspension; and
preparing a molybdenum-bismuth-based composite metal oxide represented by the following Chemical Formula 1 by drying the second catalyst suspension,
wherein the M³-based first compound is one selected from the group consisting of M³-based metal nitrates, acetates, chlorides, sulfates, and mixtures thereof,
the M³-based second compound is one selected from the group consisting of M³-based metal organic acid salts having 6 or more carbon atoms, carbonates, hydroxides, oxides, and mixtures thereof, and
the second catalyst suspension comprises a soluble M³-based compound and an insoluble M³-based compound:
[Chemical Formula 1] MOₐBi_{b}M¹_{c}M²_{d}M³ₑM⁴_{f}M⁵_{g}M⁶ₕOᵢ
in Chemical Formula 1,
Mo is molybdenum,
Bi is bismuth,
M¹ is one or more elements selected from the group consisting of V, W, P, As, Sb, S, Se, Te, Cd, and Pb,
M² is one or more elements selected from the group consisting of Fe, Cu, Zn, Cr, and Mn,
M³ is one or more elements selected from the group consisting of Co, Ni, Nb, Ta, Ga, In, Ge, and Sn,
M⁴ is one or more elements selected from the group consisting of Si, Al, Ti, Zr, and Ce,
M⁵ is one or more elements selected from the group consisting of Au, Pd, Pt, Ir, Ru, Ag, and Rh,
M⁶ is one or more elements selected from the group consisting of Na, K, Li, Rb, Cs, Ca, Mg, Sr, and Ba,
a, b, c, d, e, f, g, h, and i represent the atomic proportion of each element,
when a is 12, b is 0.01 to 20, c is 0 to 20, d is 0.001 to 15, e is 0.001 to 20, and f is 0 to 20,
g is 0 to 10, h is 0.001 to 10, and i is a numerical value determined by the oxidation state of each of the components.

2. The method of claim 1, wherein the second catalyst suspension comprises the soluble M³-based compound and the insoluble M³-based compound at a weight ratio of 1:6 to 6:1.

3. The method of claim 1, further comprising calcinating the molybdenum-bismuth-based composite metal oxide represented by Chemical Formula 1.

4. The method of claim 1, wherein Chemical Formula 1 is represented by the following Chemical Formula 2:
[Chemical Formula 2] Mo₁₂Bi_{1.5}Fe_{3.0}M³_{8.0}Cs_{0.8}
in Chemical Formula 2,
M³ is one or more elements selected from the group consisting of Co, Ni, Nb, Ta, Ga, In, Ge, and Sn.

5. The method of claim 1, further comprising heating the second catalyst suspension.

6. The method of claim 1, further comprising pulverizing the molybdenum-bismuth-based composite metal oxide represented by Chemical Formula 1.

7. The method of claim 1, further comprising supporting the molybdenum-bismuth-based composite metal oxide represented by Chemical Formula 1 on a carrier.

8. A catalyst for preparing (meth)acrylic acid prepared by the method according to any one of claims 1 to 7.

9. A method for preparing (meth)acrylic acid using a fixed bed reactor comprising a front catalyst layer and a rear catalyst layer, the method comprising:
preparing (meth)acrolein by supplying an injection gas comprising a raw material gas and oxygen gas to the front catalyst layer; and
preparing (meth)acrylic acid by supplying the (meth)acrolein to the rear catalyst layer,
wherein the catalyst for preparing (meth)acrylic acid according to claim 8 is comprised in at least one of the front catalyst layer and the rear catalyst layer.

10. The method of claim 9, wherein the raw material gas comprises one selected from the group consisting of propylene, isobutylene, t-butyl alcohol, and methyl-t-butyl ether, or a mixture thereof.
